# EUROPEAN PATENT SPECIFICATION

(11) **EP 2 400 943 B1**
(45) Date of publication and mention of the grant of the patent: **18.07.2018**
(21) Application number: 09751960.7
(22) Date of filing: 11.11.2009
(51) Int. Cl.: A61K 8/06, A61K 8/27, A61K 8/35, A61K 8/55, A61K 8/67, A61K 8/92, A61K 8/97, A61Q 19/00

(54) **COSMETIC OR PHARMACEUTICAL EMULSION FOR THE SKIN TO BE MIXED JUST PRIOR TO USE**
KOSMETISCHE ODER PHARMAZEUTISCHE ZUBEREITUNG FÜR DIE HAUT, DIE KURZ VOR DER ANWENDUNG GEMISCHT WIRD
PRÉPARATION COSMÉTIQUE OU PHARMACEUTIQUE POUR LA PEAU À MÉLANGER JUSTE AVANT L'UTILISATION

(30) Priority: 25.02.2009 GB 0903158
(43) Date of publication of application: 04.01.2012
(73) Proprietor: Taylor, Robert Peter, Little Venice London W9 1AH (GB); Lalvani, Kartar Singh, Apartment 1 London NW1 4JX (GB); Lalvani, Ajit, Oxford OX2 7ES (GB)
(72) Inventor: Taylor, Robert Peter, Little Venice London W9 1AH (GB); Lalvani, Kartar Singh, Apartment 1 London NW1 4JX (GB); Lalvani, Ajit, Oxford OX2 7ES (GB)
(86) International application number: PCT/GB2009/002642
(87) International publication number: WO 2010/097564

(56) References cited:
- GB-A- 2 453 157
- US-A- 4 438 095
- US-A- 5 925 348
- US-A- 6 149 896

## Description

### TECHNICAL FIELD

This invention concerns a cosmetic or pharmaceutical preparation intended for application to skin as a freshly prepared emulsion that can take the form of a cream or lotion.

### BACKGROUND ART

Conventional pharmaceutical or cosmetic creams or lotions, of the type that comprise both lipophilic and aqueous phases, are generally supplied ready for use in the form of stable emulsions. Such preparations include conventional moisturising creams, lotions, gels and the like. In these preparations, the lipophilic or non-polar phase usually consists of a mineral oil, such as paraffin oil or another synthetically derived material. Both the lipophilic and aqueous phases can contain small quantities of pharmaceutically active or nutrient components. Naturally sourced oils and fats, such as animal or vegetable oils and fats or lipids, are not used in such preparations, except in trace amounts, since they would rapidly degrade and become rancid, if present in significant quantities. Notwithstanding the substantial absence of natural or unsaturated oils, most conventional preparations still require the presence of preservatives for preventing both degradation of the lipophilic, or non-polar phase and the growth of mould or other organisms in the emulsion US4438095A discloses in ex. 9 a pharmaceutical preparation for application to human skin, that consists of two separate phases that are mixed: a lipid phase, the majority of which is Distilled Wheatgerm oil, and an aqua phase, the majority of which is Water.

### DISCLOSURE OF INVENTION

It is an object of the present invention to provide a formulation that uses a method of preparing a pharmaceutical or cosmetic emulsion in which the lipophilic phase comprises a significant proportion of a non-mineral oil or fat, without requiring the presence of significant quantities of preservatives or stabilizers.

In the first possible means of use, a separately prepared lipophilic component is mixed with an aqueous component to form the emulsion immediately prior to use. By the term "immediately prior to use" it is meant that the emulsion is not stored after preparation for more than a day and, preferably, is used within less than 15 minutes of preparation.

In the second possible means of use, the invention provides for a packaged pharmaceutical or cosmetic composition, comprising a lipophilic component in an openable container, suitable for use in a method in accordance with the first means, so that a measured dose of each phase can be provided in order to form the emulsion. The ratio of the two dispensed phases can be varied according to the skin type.

Preferably, the freshly formed emulsion is in the form of a cream or lotion. It is also preferred that at least one of the two phases, preferably the lipophilic or non-polar component, should include an emulsifying agent.

The two components may be provided in separate containers, or in two separate compartments of a single container. In one embodiment, the two components or phases are each contained in separate openable containers or capsules, either or both of which contain measured amounts of the components. Preferably, at least one of the components includes a measured amount of a nutrient or a pharmaceutically active component.

In preferred embodiments the lipophilic component is provided in a measured amount contained in an openable gelatin capsule and the aqueous component is provided either in an ampule or a larger dispenser. A plurality of such capsules or ampules can be packaged together with appropriate mixing instructions. Most preferably, there is no need for both phases to include a preservative, stabilizing or antibacterial agent.

It is because the aqueous and lipophilic components can be kept separately from one another, up until the time when it is wished to apply the emulsion, that a majority of the lipophilic component may comprise a natural or unsaturated oil, without there being any significant risk of this component becoming rancid, during storage. This risk is minimized when the lipophilic component is packed in a sealed capsule, or container, preferably under a nitrogen atmosphere. A further effect, also derived from the fact that the two phases need not be mixed until the emulsion is required for use, is that there is no need for any preservative or stabilizer to be included.

The advantage of avoiding the use of preservatives, synthetic chemical additives, synthetic emulsifiers and mineral oil derived components, is that certain harmful effects, that these substances may have and that are not generally associated with naturally derived substances, may be avoided. For example, skin allergies, contact dermatitis and subjective irritation can be caused by contact with mineral oil or certain preservatives. These are less likely to be provoked by contact with natural oils and nutrients.

A preparation in accordance with the present invention can be supplied in a pack of two containers, one containing the aqueous component and the other containing the lipophilic component, so that an emulsion of the two may be freshly prepared for immediate application to the skin.

Alternatively, the two phases may be supplied in two separately encapsulated portions that can be opened and mixed together to provide a single dose or application. It is intended that the two components should be mixed by the end consumer, and it is preferred that such mixing should take place freshly before each application of the preparation. Mixing can be carried out in a container, or in the palm of one hand using the finger tips of the other, or by rubbing two palms together.

The proportion of the two components used by an individual may be varied in a way which was not possible with conventionally prepared and pre-formed emulsion creams or lotions.

The emulsifying agent or agents are preferably of a natural origin and can be a lecithin, or a derivative, that is rich in phospholipid, or can be synthesized or derived from a natural source, such as glucose, or can be a modified starch emulsifier produced by a reaction between starch and a long chain fatty acid. The emulsion may be a water in oil, or oil in water emulsion.

Most preferably, the composition provides a cosmetic, such as a moisturising cream or lotion, preferably for application to hands, face or body.
The components of the lipid phase and the aqua phase are freshly combined to form an ultra fine cream before each application. Because these two phases are held separately before mixing, they can each hold a greater range and concentration of active nutrients and antioxidants than is otherwise possible.

The lipid phase is designed to include natural rejuvenating oils and lipid soluble antioxidant vitamins. It includes : Distilled Wheatgerm oil, Lecithin Emulmetik 100, DL-alpha tocopherol, Distilled Avocado oil, Cosmetic grade Calendula oil, Ascorbyl Tetraisopalmitate (Nikkon VC-IP), Liquid Silicon, Alpha-lipoic acid 99%, Jasmine oil, Vitamin A palmitate (1M/g) oily, Rose oil cosmetic grade, Coenzyme Q10 ubidecarenone, Orange oil 5 fold extract, Lavendar oil, Lutein Esters 20% trans-lutein 10%.
The aqua phase is designed to include concentrated water soluble antioxidant vitamins and actives. It includes : Liquid Panthenol, Nicotinamide, Riboflavin 5'-Phosphate sodium, Zinc Sulphate heptahydrate, D-Biotin, Aloe Life Aloe Vera gel Freeze dried powder 20, Keltrol SFT CG, Thiamine nitrate, Lemon Oil X5 fold, Dermican PW LS9838, Cremaphor PEG40 Hydrated castor oil, Potassium sorbate, De-mineralised water.

Keltrol SFT CG is Xanthan Gum, and Dermican PW LS9838 is Mannitol (and) Acetyl Tetrapeptide-9. Some of the constituents come within the classification of certain vitamins. Liquid Panthenol is a form of Vitamin B5. Nicotinamide is a form of Vitamin B3. Riboflavin 5-Phosphate Sodium is one of more than 200 forms of Vitamin B2. DL-alpha tocopherol is a form of Vitamin E. Vitamin A Palmitate is a form of Vitamin A. D-Biotin is a form of Biotin. The specific forms of these vitamins are specifically chosen because of their particular qualities relevant to the purpose of the formulation. Lemon oil and orange oil are two of the many citrus oils. Jasmine oil, Rose oil, and Lavendar oil, are all fragrant oils.

The fresh preparation method using these twin serums eliminates the need for the usual, and routinely used, petroleum based oils, paraffin waxes and heavy preservatives that block pores, restrict oxygen flow, and cause a dullening of the skin. Instead, it utilises a distilled Wheatgerm, Avocado and Calendula oil base, enriched with nutrient complexes including, Jasmine, Lavender and Citrus oils.

As we age, skin gradually loses its elasticity and the production of natural skin oils decreases, making it appear thinner and more lifeless. The twin serums actively replenish, with a combination of advanced skin micronutrients plus natural, aromatic oils including Calendula, Wheatgerm, Avocado, Jasmine, Lavender and Orange oils. These develop a cell hydrating, calming sensation and, when used last thing, prepare for a deep night's sleep.

As skin cells age, their normal cellular function and collagen production declines leaving them vulnerable and in need of specialist support. The twin serums provide nourishing B-complex vitamins, vitamin C, natural source silica, vitamin E, Biotin, Aloe Vera and Co-enzyme Q10 and other micronutrients that help boost the natural production of collagen and elastin. Both of these are essential factors in retaining supple skin and a youthful appearance. Silica is well known for its role as a "beauty mineral" and is vital for the strength and functioning of all connective tissues, and especially in the skin where it holds in moisture. These powerful ingredients work together to revitalise tired skin cells, leaving the complexion looking vibrant, firmer and more defined.

With time, everyone's skin's renewal process slows down leading to reduced cell regeneration and a breakdown in collagen structure. The antioxidants fight free radicals, which are the major cause of premature ageing and wrinkle formation, and help prevent oxidative damage of skin cells. Vitamin C supports the formation of collagen which underpins skin structure and counteracts wrinkle formation, whilst Vitamin E helps neutralise free radicals and moisturise the skin.

Ultraviolet radiation in sunlight and pollution generates free radicals which can damage cells including their genetic structure (DNA), to rapidly accelerate premature skin ageing. The formulation provides a synergistic combination of powerful water and oil soluble antioxidants including 'the universal antioxidants Alpha Lipoic Acid (ALA), vitamin A, natural source vitamin E, lutein and a special lipid soluble vitamin C. The antioxidants and micronutrients in the twin serum help to protect skin cells and dermal proteins including collagen and elastin, helping to keep skin looking smooth and reduce wrinkle lines.
The formulation includes the biological antioxidant Coenzyme-Q 10, which supports the skin's metabolism from within to continuously fight the appearance of wrinkles.

The aqueous phase can also contain a small amount of hydrolysed vegetable protein or an amino acid or acids derived therefrom, which are known for their moisture retention and skin firming properties.
The most preferred oil is a self emulsifying oil, such as Wheatgerm oil, which will form an emulsion with water, without requiring the presence of a separate emulsifying agent. The emulsifying agent, or agents, when used, are preferably derived from natural sources, such as purified lecithin, which is rich in phospholipids, or a glucose derivative.
Unlike the mineral oils, usually employed in ready formed emulsions, the natural oil or fat, used in the present invention, is more readily absorbed into the skin and, therefore, can carry nutrients or pharmaceutically active materials deeper and more rapidly into the stratum corneum. Also, the material can provide a softer and smoother texture to the hydrophilic keratin layer of the skin. The phospholipids in lecithin can help reduce the loss of water from the epidermis.
The following is the optimal formulation -
An aqua phase that consists of :
∼ 88% De-mineralised water
4% Liquid Panthenol
4% Nicotinamide
0.00125% Riboflavin 5'- Phosphate sodium
0.4% Zinc Sulphate heptahydrate
0.01% D-Biotin
1% Aloe Life Aloe Vera gel Freeze dried powder 20
0.07% Keltrol SFT CG
0.5% Thiamine nitrate
0.2% Lemon Oil X5 fold
1% Dermican PW LS9838
0.56% Cremaphor PEG40 Hydrated castor oil
0.12% Potassium sorbate
and an Oil Phase that consists of :
∼ 55% Distilled Wheatgerm oil
13.50% Lecithin Emulmetik 100
10.80% DL-alpha tocopherol
10.80% Distilled Avocado oil
5.40% Cosmetic grade Calendula oil
2.00% Ascorbyl Tetraisopalmitate (Nikkon VC-IP)
0.90% Liquid Silicon
0.50% Alpha-lipoic acid 99%
0.20% Jasmine oil
0.40% Vitamin A palmitate (1M/g) oily
0.12% Rose oil cosmetic grade
0.10% Coenzyme Q10 ubidecarenone
0.10% Orange oil 5 fold extract
0.076% Lavendar oil
0.02% Lutein Esters 20% (trans-lutein 10%)

The following is the preferred range of proportions :

### The aqua phase:

Liquid Panthenol - 0.1% to 20%
Nicotinamide - 0.1% to 20%
Riboflavin 5'- Phosphate sodium - 0.0001% to 2%
Zinc Sulphate heptahydrate - 0.01% to 10%
D-Biotin - 0.001% to 2%
Aloe Life Aloe Vera gel Freeze dried powder 20 - 0.01% to 20%
Keltrol SFT CG - 0.007% to 0.7%
Thiamine nitrate - 0.05% to 5%
Lemon Oil X5 fold - 0.02% to 2%
Dermican PW LS9838 - 0.1% to 10%
Cremaphor PEG40 Hydrated castor oil - 0.056% to 5.6%
Potassium sorbate - 0.012% to 1.2%

### The Oil Phase :

Lecithin Emulmetik 100 - 2% to 40%
DL-alpha tocopherol - 1% to 25%
Distilled Avocado oil - 1% to 40%
Cosmetic grade Calendula oil - 1% to 30%
Ascorbyl Tetraisopalmitate (Nikkon VC-IP) - 0.1% to 20%
Liquid Silicon - 2% to 30%
Alpha-lipoic acid 99% - 0.01% to 5%
Jasmine oil - 0.01% to 5%
Vitamin A palmitate (1M/g) oily - 0.0015% to 0.3%
Rose oil cosmetic grade - 0.01% to 2%
Coenzyme Q10 ubidecarenone - 0.01% to 10%
Orange oil 5 fold extract - 0.01% to 5%
Lavendar oil - 0.01% to 5%
Lutein Esters 20% (trans-lutein 10%) - 0.001% to 2%

In total, the composition when used in these proportions does not comprise per litre more than 300mg of amino acids in a protein or amino acid complex form.
The ratio of the two dispensed Aqua and Lipid phases can be varied according to skin type. The present invention is as defined in Claim 1. The composition can be more specifically for use with wrinkles, scars, anti-aging, blemishes, skin repair and healing.

### Directions for use :

Dispense 1 pump of lipid serum (smaller bottle) and 2 pumps of aqua serum (larger bottle) into the cupped palm of the hand. Using the index finger, mix the two together in the palm for about 5 seconds, until a light creamy yellow coloured serum develops. Using the index finger, first apply some of this serum to areas of most concern, such as deeper expression lines and around your closed eyes. Then add 1 further pump of aqua serum to the remaining serum in your palm. Rub your two palms briskly together for a few more seconds, so the mixture is spread between your palms. Apply this lighter mixture directly from your two palms evenly around the whole face and closed eyes. Repeat the process as required, if desired applying to the neck and back of hands too. Allow about 10 minutes for full penetration of the active materials before applying makeup. The lightness of this unique elixir allows your skin to breathe, so it can be used twice a day, in the morning and at night.

### INDUSTRIAL APPLICABILITY

This invention concerns a cosmetic or pharmaceutical preparation intended for application to skin as a freshly prepared emulsion that can take the form of a cream or lotion. In its optimal form there is an Aqua Phase that consists of : 4% Liquid Panthenol, 4% Nicotinamide, 0.00125% Riboflavin 5'- Phosphate sodium, 0.4% Zinc Sulphate heptahydrate, 0.01% D-Biotin, 1% Aloe Life Aloe Vera gel Freeze dried powder 20, 0.07% Keltrol SFT CG, 0.5% Thiamine nitrate, 0.2% Lemon Oil X5 fold, 1% Dermican PW LS9838, 0.56% Cremaphor PEG40 Hydrated castor oil, 0.12% Potassium sorbate, and approximately 88% De-mineralised water; and an Oil Phase that consists of : 13.50% Lecithin Emulmetik 100, 10.80% DL-alpha tocopherol, 10.80% Distilled Avocado oil, 5.40% Cosmetic grade Calendula oil, 2.00% Ascorbyl Tetraisopalmitate (Nikkon VC-IP), 0.90% Liquid Silicon, 0.50% Alpha-lipoic acid 99%, 0.20% Jasmine oil, 0.40% Vitamin A palmitate (1M/g) oily, 0.12% Rose oil cosmetic grade, 0.10% Coenzyme Q10 ubidecarenone, 0.10% Orange oil 5 fold extract, 0.076% Lavendar oil, 0.02% Lutein Esters 20% (trans-lutein 10%), and approximately 55% Distilled Wheatgerm oil.

## Claims

1. A cosmetic or pharmaceutical preparation intended for application to human skin, that consists of two separate phases that are mixed just prior to use : a lipid phase, the majority of which is Distilled Wheatgerm oil, and which also comprises Lecithin, Fragrant oils, Citrus oil, Vitamin A, Vitamin C, Vitamin E, Coenzyme Q10, Avocado oil, Calendula oil, Silicon, Lipoic acid, Lutein; and an aqua phase, the majority of which is Water, and which also comprises B vitamins, Zinc, Aloe Vera, Citrus Oil, Mannitol (and) Acetyl Tetrapeptide-9 (Dermican PW LS9838), PEG40 Hydrated Castor Oil (Cremaphor), Sorbic acid.

2. A cosmetic or pharmaceutical preparation according to Claim 1 in which the lipid phase comprises Lecithin Emulmetik 100, DL-alpha tocopherol, Distilled Avocado oil, Cosmetic grade Calendula oil, Ascorbyl Tetraisopalmitate (Nikkon VC-IP), Liquid Silicon, Alpha-lipoic acid 99%, Vitamin A palmitate (1M/g) oily, Rose oil cosmetic grade and Jasmine oil and Lavendar oil, Coenzyme Q10 ubidecarenone, Orange oil 5 fold extract, Lutein Esters 20% trans-lutein 10%; and an aqua phase, the majority of which is De-mineralized water, and which also comprises Liquid Panthenol and Nicotinamide and Riboflavin 5'-Phosphate sodium and Thiamine nitrate and D-Biotin, Zinc Sulphate heptahydrate, Aloe Life Aloe Vera gel Freeze dried powder 20, Xanthan Gum (Keltrol SFT CG), Lemon Oil X5 fold, Mannitol (and) Acetyl Tetrapeptide-9 (Dermican PW LS9838), Cremaphor PEG40 Hydrated castor oil, Potassium sorbate.

3. A cosmetic or pharmaceutical preparation according to claim 2 in which the preferred range of percentages of the lipid phase are : 2% to 40% Lecithin Emulmetik 100, 1% to 25% DL-alpha tocopherol, 1% to 40% Distilled Avocado oil, 1% to 30% Cosmetic grade Calendula oil, 0.1% to 20% Ascorbyl Tetraisopalmitate (Nikkon VC-IP), 2% to 30% Liquid Silicon, 0.01% to 5% Alpha-lipoic acid 99%, 0.01% to 5% Jasmine oil, 0.0015% to 0.3% Vitamin A palmitate (1M/g) oily, 0.01% to 2% Rose oil cosmetic grade, 0.01% to 10% Coenzyme Q10 ubidecarenone, 0.01% to 5% Orange oil 5 fold extract, 0.01% to 5% Lavendar oil, 0.001% to 2% Lutein Esters 20% (trans-lutein 10%); and in which the preferred range of the percentages of the Aqua phase are : 0.1% to 20% Liquid Panthenol, 0.1% to 20% Nicotinamide, 0.0001% to 2% Riboflavin 5'- Phosphate sodium, 0.01% to 10% Zinc Sulphate heptahydrate, 0.001% to 2% D-Biotin, 0.01% to 20% Aloe Life Aloe Vera gel Freeze dried powder 20, 0.007% to 0.7% Xanthan Gum (Keltrol SFT CG), 0.05% to 5% Thiamine nitrate, 0.02% to 2% Lemon Oil X5 fold, 0.1% to 10% Mannitol (and) Acetyl Tetrapeptide-9 (Dermican PW LS9838), 0.056% to 5.6% Cremaphor PEG40 Hydrated castor oil, 0.012% to 1.2% Potassium sorbate.

4. A cosmetic or pharmaceutical preparation according to claim 3 in which the approximate percentages of the lipid phase are : 13.50% Lecithin Emulmetik 100, 10.80% DL-alpha tocopherol, 10.80% Distilled Avocado oil, 5.40% Cosmetic grade Calendula oil, 2.00% Ascorbyl Tetraisopalmitate (Nikkon VC-IP), 0.90% Liquid Silicon, 0.50% Alpha-lipoic acid 99%, 0.20% Jasmine oil, 0.40% Vitamin A palmitate (1M/g) oily, 0.12% Rose oil cosmetic grade, 0.10% Coenzyme Q10 ubidecarenone, 0.10% Orange oil 5 fold extract, 0.076% Lavendar oil, 0.02% Lutein Esters 20% (trans-lutein 10%), and approximately 55% Distilled Wheatgerm oil; and in which the approximate percentages of the Aqua phase are : 4% Liquid Panthenol, 4% Nicotinamide, 0.00125% Riboflavin 5'- Phosphate sodium, 0.4% Zinc Sulphate heptahydrate, 0.01% D-Biotin, 1% Aloe Life Aloe Vera gel Freeze dried powder 20, 0.07% Xanthan Gum (Keltrol SFT CG), 0.5% Thiamine nitrate, 0.2% Lemon Oil X5 fold, 1% Mannitol (and) Acetyl Tetrapeptide-9 (Dermican PW LS9838), 0.56% Cremaphor PEG40 Hydrated castor oil, 0.12% Potassium sorbate, and approximately 88% De-mineralised water.

5. A cosmetic or pharmaceutical preparation according to any one of the preceding claims in which the ratio of the two dispensed Aqua and Lipid phases can be varied according to skin type.

6. A cosmetic preparation according to any one of the preceding claims, which is specifically for use with wrinkles, scars, anti-aging, blemishes,

7. A pharmaceutical preparation according to Claims 1-5 for use as a medicament in skin repair and healing.

8. A cosmetic or pharmaceutical preparation according to any one of the claims, in which preservatives are not included in the composition.

9. A cosmetic or pharmaceutical preparation according to any one of the claims in which the composition includes one or more carriers or excipients.

10. A cosmetic or pharmaceutical preparation according to any one of the claims wherein the composition consists essentially of the components defined in that claim.

## Patentansprüche

1. Kosmetische oder pharmazeutische Zubereitung, die für die Anwendung auf menschliche Haut vorgesehen ist und die aus zwei getrennten Phasen besteht, die unmittelbar vor der Verwendung gemischt werden: eine Lipidphase, deren Großteil destilliertes Weizenkeimöl ist und die auch Lecithin, Duftöle, Zitrusöl, Vitamin A, Vitamin C, Vitamin E, Coenzym Q10, Avocadoöl, Calendulaöl, Silicon, Liponsäure, Lutein umfasst; und eine wässrige Phase, deren Großteil Wasser ist und die auch B-Vitamine, Zink, Aloe Vera, Zitrusöl, Mannit (und) Acetyltetrapeptid-9 (Dermican PW LS9838), PEG40 hydriertes Castoröl (Cremaphor), Sorbinsäure umfasst.

2. Kosmetische oder pharmazeutische Zubereitung gemäß Anspruch 1, wobei die Lipidphase Lecithin Emulmetik 100, DL-alpha-Tocopherol, destilliertes Avocadoöl, Calendulaöl kosmetischer Qualität, Ascorbyltetraisopalmitat (Nikkon VC-IP), flüssiges Silicon, alpha-Liponsäure 99 %, Vitamin-A-palmitat (1 M/g) ölig, Rosenöl kosmetischer Qualität und Jasminöl und Lavendelöl, Coenzym Q10 Ubidecarenon, Orangenöl 5-fach-Extrakt, Luteinester 20 % trans-Lutein 10 % umfasst; und eine wässrige Phase, deren Großteil entmineralisiertes Wasser ist und die auch flüssiges Panthenol und Nicotinamid und Riboflavin-5'-phosphat-Natrium und Thiaminnitrat und D-Biotin, Zinksulfatheptahydrat, Aloe Life Aloe-Vera-Gel gefriergetrocknetes Pulver 20, Xanthangummi (Keltrol SFT CG), Zitronenöl X5-fach, Mannit (und) Acetyltetrapeptid-9 (Dermican PW LS9838), Cremaphor PEG40 hydriertes Castoröl, Kaliumsorbat umfasst.

3. Kosmetische oder pharmazeutische Zubereitung gemäß Anspruch 2, wobei die bevorzugten Bereiche der Prozentanteile der Lipidphase sind: 2 % bis 40 % Lecithin Emulmetik 100, 1 % bis 25 % DL-alpha-Tocopherol, 1 % bis 40 % destilliertes Avocadoöl, 1 % bis 30 % Calendulaöl kosmetischer Qualität, 0,1 % bis 20 % Ascorbyltetraisopalmitat (Nikkon VC-IP), 2 % bis 30 % flüssiges Silicon, 0,01 % bis 5 % alpha-Liponsäure 99 %, 0,01 % bis 5 % Jasminöl, 0,0015 % bis 0,3 % Vitamin-A-palmitat (1 M/g) ölig, 0,01 % bis 2 % Rosenöl kosmetischer Qualität, 0,01 % bis 10 % Coenzym Q10 Ubidecarenon, 0,01 % bis 5 % Orangenöl 5-fach-Extrakt, 0,01 % bis 5 % Lavendelöl, 0,001 % bis 2 % Luteinester 20 % (trans-Lutein 10 %); und wobei die bevorzugten Bereiche der Prozentanteile der wässrigen Phase sind: 0,1 % bis 20 % flüssiges Panthenol, 0,1 % bis 20 % Nicotinamid, 0,0001 % bis 2 % Riboflavin-5'-phosphat-Natrium, 0,01 % bis 10 % Zinksulfatheptahydrat, 0,001 % bis 2 % D-Biotin, 0,01 % bis 20 % Aloe Life Aloe-Vera-Gel gefriergetrocknetes Pulver 20, 0,007 % bis 0,7 % Xanthangummi (Keltrol SFT CG), 0,05 % bis 5 % Thiaminnitrat, 0,02 % bis 2 % Zitronenöl X5-fach, 0,1 % bis 10 % Mannit (und) Acetyltetrapeptid-9 (Dermican PW LS9838), 0,056 % bis 5,6 % Cremaphor PEG40 hydriertes Castoröl, 0,012 % bis 1,2 % Kaliumsorbat.

4. Kosmetische oder pharmazeutische Zubereitung gemäß Anspruch 3, wobei die ungefähren Prozentanteile der Lipidphase sind: 13,50 % Lecithin Emulmetik 100, 10,80 % DL-alpha-Tocopherol, 10,80 % destilliertes Avocadoöl, 5,40 % Calendulaöl kosmetischer Qualität, 2,00 % Ascorbyltetraisopalmitat (Nikkon VC-IP), 0,90 % flüssiges Silicon, 0,50 % alpha-Liponsäure 99 %, 0,20 % Jasminöl, 0,40 % Vitamin-A-palmitat (1 M/g) ölig, 0,12% Rosenöl kosmetischer Qualität, 0,10 % Coenzym Q10 Ubidecarenon, 0,10 % Orangenöl 5-fach-Extrakt, 0,076 % Lavendelöl, 0,02 % Luteinester 20 % (trans-Lutein 10 %) und etwa 55 % destilliertes Weizenkeimöl; und wobei die ungefähren Prozentanteile der wässrigen Phase sind: 4 % flüssiges Panthenol, 4 % Nicotinamid, 0,00125 % Riboflavin-5'-phosphat-Natrium, 0,4 % Zinksulfatheptahydrat, 0,01 % D-Biotin, 1 % Aloe Life Aloe-Vera-Gel gefriergetrocknetes Pulver 20, 0,07 % Xanthangummi (Keltrol SFT CG), 0,5 % Thiaminnitrat, 0,2 % Zitronenöl X5-fach, 1 % Mannit (und) Acetyltetrapeptid-9 (Dermican PW LS9838), 0,56 % Cremaphor PEG40 hydriertes Castoröl, 0,12 % Kaliumsorbat und etwa 88 % entmineralisiertes Wasser.

5. Kosmetische oder pharmazeutische Zubereitung gemäß einem der vorstehenden Ansprüche, wobei das Verhältnis der beiden ausgegebenen wässrigen und Lipidphasen dem Hauttyp entsprechend variiert werden kann.

6. Kosmetische Zubereitung gemäß einem der vorstehenden Ansprüche, die spezifisch für die Verwendung bei Falten, Narben, zum Anti-Aging, bei Hautmakeln ist.

7. Pharmazeutische Zubereitung gemäß Ansprüchen 1-5 für die Verwendung als Medikament bei der Hautreparatur und -heilung.

8. Kosmetische oder pharmazeutische Zubereitung gemäß einem der Ansprüche, wobei in der Zusammensetzung keine Konservierungsmittel enthalten sind.

9. Kosmetische oder pharmazeutische Zubereitung gemäß einem der Ansprüche, wobei die Zusammensetzung einen oder mehrere Träger oder Hilfsstoffe enthält.

10. Kosmetische oder pharmazeutische Zubereitung gemäß einem der Ansprüche, wobei die Zusammensetzung im Wesentlichen aus den in dem Anspruch definierten Komponenten besteht.

## Revendications

1. Préparation cosmétique ou pharmaceutique prévue pour une application à la peau humaine, qui est constituée de deux phases distinctes qui sont mélangées juste avant l'utilisation : une phase lipidique, dont la majorité est constituée d'huile de germe de blé distillée, et qui comprend également de la lécithine, des huiles parfumées, de l'huile d'agrumes, de la vitamine A, de la vitamine C, de la vitamine E, du coenzyme Q10, de l'huile d'avocat, de l'huile de calendula, du silicium, de l'acide lipoïque, de la lutéine ; et une phase aqueuse, dont la majorité est constituée d'eau, et qui comprend également des vitamines B, du zinc, de l'aloe vera, de l'huile d'agrumes, du mannitol (et) de l'acétyl-tétrapeptide-9 (Dermican PW LS9838), de l'huile de ricin hydratée PEG40 (Cremaphor), de l'acide sorbique.

2. Préparation cosmétique ou pharmaceutique selon la revendication 1, dans laquelle la phase lipidique comprend de la lécithine Emulmetik 100, du DL-alpha-tocophérol, de l'huile d'avocat distillée, de l'huile de calendula de qualité cosmétique, du tétraisopalmitate d'ascorbyle (Nikkon VC-IP), du silicium liquide, de l'acide alpha-lipoïque à 99%, du palmitate de vitamine A (1M/g) huileux, de l'huile de rose de qualité cosmétique et de l'huile de jasmin et de l'huile de lavande, du coenzyme Q10 ubidécarénone, de l'huile d'orange extraite 5 fois, des esters de lutéine à 20%, trans-lutéine à 10% ; et une phase aqueuse, dont la majorité est constituée d'eau déminéralisée, et qui comprend également du panthénol liquide et du nicotinamide et de la riboflavine 5'-phosphate de sodium et du nitrate de thiamine et de la D-biotine, du sulfate de zinc heptahydraté, du gel d'aloe vera Aloe Life, poudre lyophilisée 20, de la gomme xanthane (Keltrol SFT CG), de l'huile de citron x5, du mannitol (et) de l'acétyl-tétrapeptide-9 (Dermican PW LS9838), de l'huile de ricin hydratée PEG40 Cremaphor, du sorbate de potassium.

3. Préparation cosmétique ou pharmaceutique selon la revendication 2, dans laquelle la plage préférée de pourcentages de la phase lipidique est : de 2% à 40% de lécithine Emulmetik 100, de 1% à 25% de DL-alpha-tocophérol, de 1% à 40% d'huile d'avocat distillée, de 1% à 30% d'huile de calendula de qualité cosmétique, de 0,1% à 20% de tétraisopalmitate d'ascorbyle (Nikkon VC-IP), de 2% à 30% de silicium liquide, de 0,01% à 5% d'acide alpha-lipoïque à 99%, de 0,01% à 5% d'huile de jasmin, de 0,0015% à 0,3% de palmitate de vitamine A (1M/g) huileux, de 0,01% à 2% d'huile de rose de qualité cosmétique, de 0,01% à 10% de coenzyme Q10 ubidécarénone, de 0,01% à 5% d'huile d'orange extraite 5 fois, de 0,01% à 5% d'huile de lavande, de 0,001% à 2% d'esters de lutéine à 20% (trans-lutéine à 10%) ; et où la plage préférée de pourcentages de la phase aqueuse est : de 0,1% à 20% de panthénol liquide, de 0,1% à 20% de nicotinamide, de 0,0001% à 2% de riboflavine 5'-phosphate de sodium, de 0,01% à 10% de sulfate de zinc heptahydraté, de 0,001% à 2% de D-biotine, de 0,01% à 20% de gel d'aloe vera Aloe Life, poudre lyophilisée 20, de 0,007% à 0,7% de gomme xanthane (Keltrol SFT CG), de 0,05% à 5% de nitrate de thiamine, de 0,02% à 2% d'huile de citron x5, de 0,1% à 10% de mannitol (et) d'acétyl-tétrapeptide-9 (Dermican PW LS9838), de 0,056% à 5,6% d'huile de ricin hydratée PEG40 Cremaphor, de 0,012% à 1,2% de sorbate de potassium.

4. Préparation cosmétique ou pharmaceutique selon la revendication 3, dans laquelle les pourcentages approximatifs de la phase lipidique sont : de 13,50% de lécithine Emulmetik 100, de 10,80% de DL-alpha-tocophérol, de 10,80% d'huile d'avocat distillée, de 5,40% d'huile de calendula de qualité cosmétique, de 2,00% de tétraisopalmitate d'ascorbyle (Nikkon VC-IP), de 0,90% de silicium liquide, de 0,50% d'acide alpha-lipoïque à 99%, de 0,20% d'huile de jasmin, de 0,40% de palmitate de vitamine A (1M/g) huileux, de 0,12% d'huile de rose de qualité cosmétique, de 0,10% de coenzyme Q10 ubidécarénone, de 0,10% d'huile d'orange extraite 5 fois, de 0,076% d'huile de lavande, de 0,02% d'esters de lutéine à 20% (trans-lutéine à 10%) et environ 55% d'huile de germe de blé distillée ; et où les pourcentages approximatifs de la phase aqueuse sont : de 4% de panthénol liquide, de 4% de nicotinamide, de 0,00125% de riboflavine 5'-phosphate de sodium, de 0,4% de sulfate de zinc heptahydraté, de 0,01% de D-biotine, de 1% de gel d'aloe vera Aloe Life, poudre lyophilisée 20, de 0,07% de gomme xanthane (Keltrol SFT CG), de 0,5% de nitrate de thiamine, de 0,2% d'huile de citron x5, de 1% de mannitol (et) d'acétyl-tétrapeptide-9 (Dermican PW LS9838), de 0,56% d'huile de ricin hydratée PEG40 Cremaphor, de 0,12% de sorbate de potassium, et environ 88% d'eau déminéralisée.

5. Préparation cosmétique ou pharmaceutique selon l'une quelconque des revendications précédentes, dans laquelle le rapport des deux phases aqueuse et lipidique distribuées peut être varié selon le type de peau.

6. Préparation cosmétique ou pharmaceutique selon l'une quelconque des revendications précédentes, qui est destinée spécifiquement à une utilisation pour les rides, les cicatrices, comme antivieillissement, pour les imperfections.

7. Préparation pharmaceutique selon les revendications 1-5, pour une utilisation comme médicament destiné à réparer et guérir la peau.

8. Préparation cosmétique ou pharmaceutique selon l'une quelconque des revendications, dans laquelle des conservateurs ne sont pas inclus dans la composition.

9. Préparation cosmétique ou pharmaceutique selon l'une quelconque des revendications, dans laquelle la composition comporte un ou plusieurs véhicules ou excipients.

10. Préparation cosmétique ou pharmaceutique selon l'une quelconque des revendications, dans laquelle la composition est constituée essentiellement des composants définis selon cette revendication.
